(19) Europäisches Patentamt
European Patent Office
Office européen des brevets

(11) Numéro de publication : **0 459 854 A1**

# (12) DEMANDE DE BREVET EUROPEEN

(21) Numéro de dépôt : **91401242.2**

(22) Date de dépôt : **14.05.91**

(51) Int. Cl.[5] : **C22B 3/00,** G21C 19/46, G21C 19/50, C07C 321/14

(30) Priorité : **15.05.90 FR 9006041**

(43) Date de publication de la demande : **04.12.91 Bulletin 91/49**

(84) Etats contractants désignés : **BE DE ES FR GB IT SE**

(71) Demandeur : **COGEMA (COMPAGNIE GENERALE DES MATIERES NUCLEAIRES)**
**2, rue Paul Dautier B.P. 4**
**F-78141 Velizy-Villacoublay Cédex (FR)**

(72) Inventeur : **Guy, Alain**
**1 rue Urgons**
**F-77135 Pontcarré (FR)**
Inventeur : **Lemaire, Marc**
**23 rue Château Gaillard**
**F-69100 Villeurbanne (FR)**
Inventeur : **Foos, Jacques**
**33 rue Louis Scocard**
**F-91400 Orsay (FR)**
Inventeur : **Le Buzit, Gérard**
**14 Avenue Aristide Briand**
**F-91560 Crosne (FR)**
Inventeur : **Guyon, Vincent**
**235 rue Saint Martin**
**F-75003 Paris (FR)**
Inventeur : **Moutarde, Thierry**
**Route d'Uchaux (Chez L.Perez), Chemin Vicinal 4**
**F-84420 Piolenc (FR)**
Inventeur : **Chomel, Rodolphe**
**328 Avenue de Champlain**
**F-84100 Orange (FR)**
Inventeur : **Draye, Micheline**
**44 Avenue de Chambéry**
**F-73190 Challes-Les-Eaux (FR)**

(74) Mandataire : **Mongrédien, André et al**
**c/o BREVATOME 25, rue de Ponthieu**
**F-75008 Paris (FR)**

## (54) Utilisation de ligands thio-éthers pour séparer le palladium de solutions aqueuses, en particulier de solutions nitriques de dissolution d'éléments combustibles nucléaires irradiés.

(57) L'invention concerne l'utilisation de ligands thio-éthers répondant à la formule :

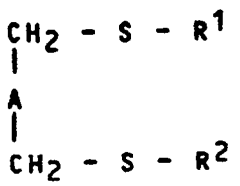
(I) ou
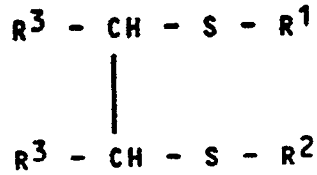
(II)

dans lesquelles $R^1$ et $R^2$ qui peuvent être identiques ou différents, représentent des radicaux alkyle, $R^3$ est un radical alkyle et A représente un radical bivalent choisi parmi les radicaux de formule :

$-(CH_2)_m-$
$-(CH_2)_n-X-(CH_2)_p$
-CH-
$R^3$

dans lesquelles m est égal à 0 ou est un nombre entier de 1 à 6, n et p sont des nombres entiers allant de 1 à 6 et X représente 0 ou S,

pour récupérer le palladium présent dans une solution aqueuse nitrique ($A_0$)de dissolution d'éléments combustibles nucléaires irradiés.

A titre d'exemple, on peut utiliser le ligand de formule (I) avec A représentant $CH_2$ - S - $CH_2$ et $R^1$ et $R^2$ représentant $C_{10}$ $H_{21}$.

EP 0 459 854 A1

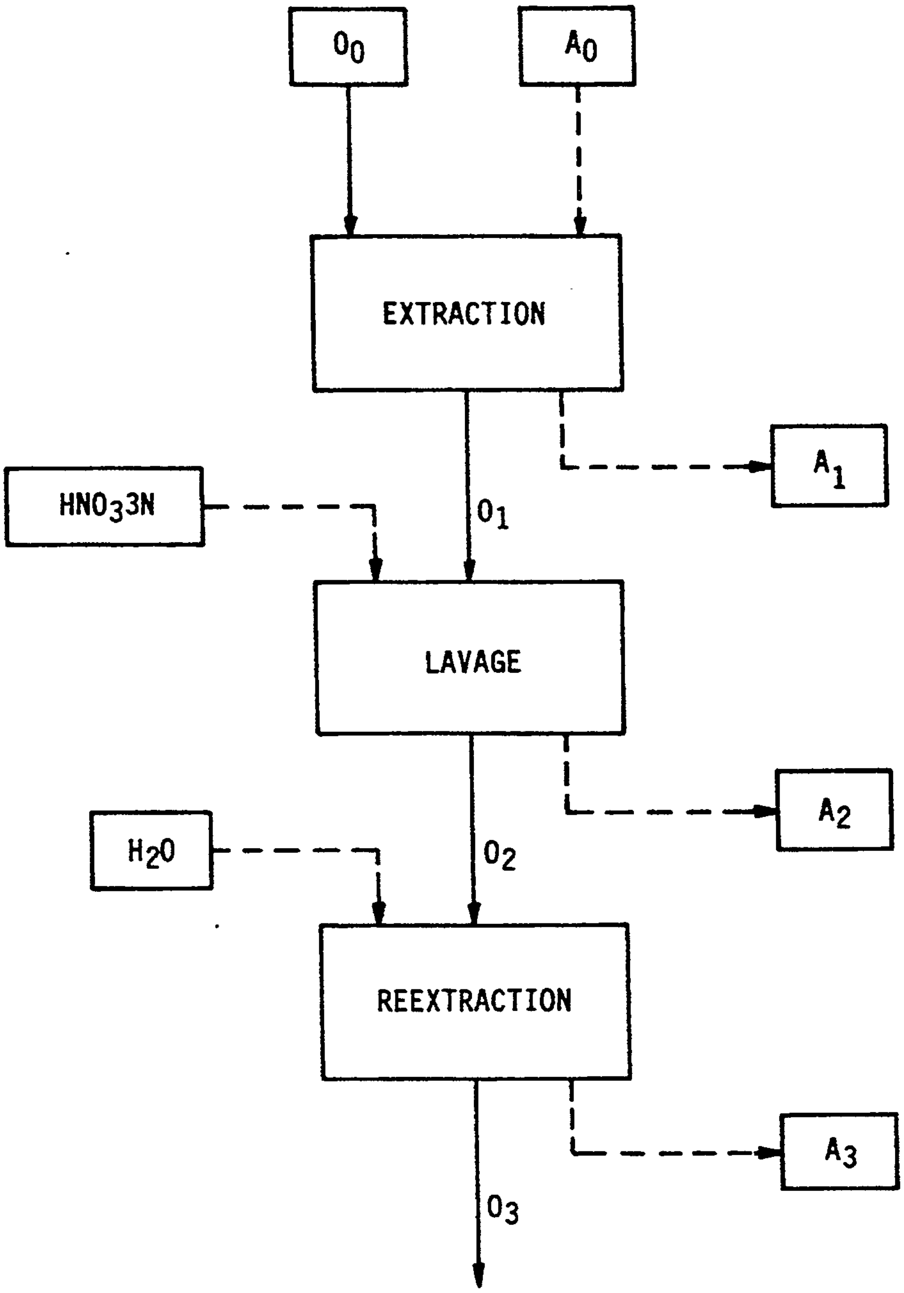

$O_0$
$A_0$
EXTRACTION
$A_1$
$HNO_3$ 3N
$O_1$
LAVAGE
$A_2$
$H_2O$
$O_2$
REEXTRACTION
$A_3$
$O_3$


FIG. 3

La présente invention concerne l'utilisation de ligands thio-éthers qui sont des thioéthers non cycliques ou podands, pour séparer le palladium de solutions aqueuses.

De façon plus précise, elle concerne des ligands thio-éthers capables de séparer sélectivement le palladium d'une solution aqueuse de retraitement de combustibles nucléaires irradiés.

Depuis plusieurs années, la technique la plus largement utilisée pour réaliser le retraitement des combustibles nucléaires irradiés consiste à dissoudre le combustible dans une solution nitrique, ce qui conduit à l'obtention d'une solution aqueuse nitrique contenant de l'uranium, du plutonium et des produits de fission. Après cette étape de dissolution, on met la solution aqueuse nitrique ainsi obtenue en contact avec un solvant organique pour extraire dans celui-ci l'uranium et le plutonium et les séparer de la majorité des produits de fission. On réextrait ensuite l'uranium et le plutonium dans une phase aqueuse puis on sépare l'uranium du plutonium présents dans cette phase aqueuse au moyen d'un solvant organique.

Parmi les produits de fission présents dans la solution aqueuse nitrique de dissolution, on trouve du palladium en concentration non négligeable, qu'il serait intéressant de récupérer à ce stade.

Cependant, pour séparer le palladium de cette solution, il est nécessaire de disposer d'un moyen permettant d'extraire sélectivement le palladium en présence de nombreux éléments dont l'uranium et le plutonium qui se trouvent dans cette solution à des concentrations beaucoup plus importantes que le palladium.

La présente invention a précisément pour objet un procédé pour séparer le palladium présent dans une solution aqueuse au moyen de ligands thio-éthers qui sont des extractants sélectifs du palladium, ayant une bonne stabilité sous irradiation et qui permettent de séparer quantitativement le palladium d'une solution de dissolution de combustibles nucléaires irradiés.

Selon l'invention, ce procédé comprend les étapes suivantes :

a) mettre en contact la solution aqueuse contenant le palladium avec un extractant organique constitué par un thioéther répondant à la formule:

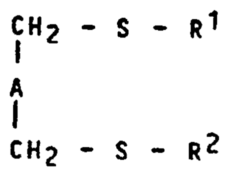
(I) ou
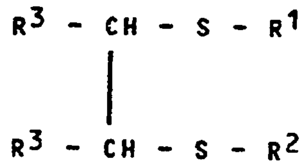
(II)

ou comprenant un radical de formule :

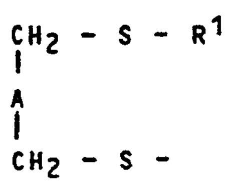
(Ia) ou
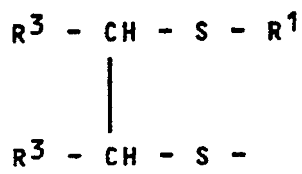
(II a)

dans lesquelles $R^1$ et $R^2$ qui peuvent être identiques ou différents, représentent des radicaux alkyle, $R^3$ représente un radical alkyle et A représente un radical bivalent choisi parmi les radicaux de formule :

$-(CH_2)_m-$

$-(CH_2)_n-X-(CH_2)_p,$

-CH-

$R^3$

dans lesquelles m est égal à 0 ou est un nombre entier allant de 1 à 6, n et p sont des nombres entiers allant de 1 à 6 et X représente 0 ou S, pour complexer le palladium avec cet extractant, et

b) séparer la solution aqueuse appauvrie en palladium du complexe de palladium formé dans l'étape a).

Les radicaux alkyle utilisés dans l'invention pour $R^1$, $R^2$ et $R^3$ peuvent être des radicaux linéaires ou ramifiés. Ils ont de préférence de 4 à 18 atomes de carbone pour conférer au ligand une forte lipophilicité qui favorise l'extraction du palladium.

A titre d'exemple de ligands thio-éthers particulièrement intéressants, on peut citer les composés suivants :

Le trithia-6,9,12 heptadécane :

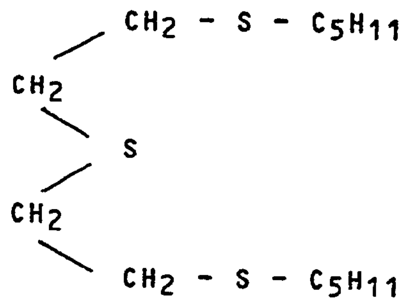

Composé n° 1

Le trithia-11,14,17 heptacosane :

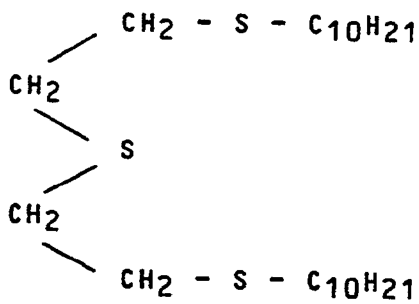

Composé n° 2

Le dithia-6,10 pentadécane :

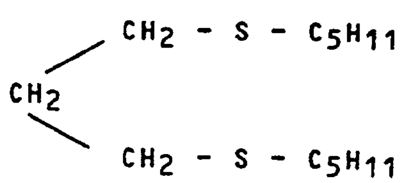

Composé n° 3

Les ligands thio-éthers de l'invention peuvent être préparés par des procédés classiques à partir des dithiolates de sodium correspondants de formule $NaSCH_2ACH_2SNa$ ou de formule :

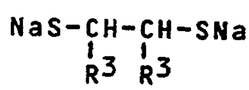

que l'on fait réagir avec les dérivés halogénés de formule $R^1X$ et $R^2X$ dans lesquels $R^1$ et $R^2$ ont la signification donnée ci-dessus et X est un atome d'halogène. Ceci correspond au schéma réactionnel suivant :

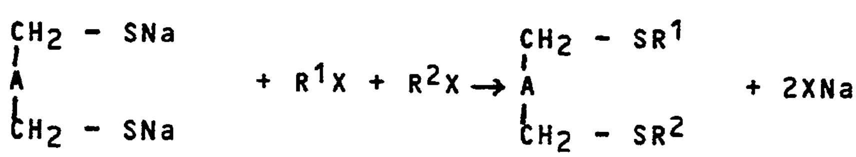

Cette réaction peut être effectuée dans un mélange d'éthanol et d'éthanolate de sodium.

Le dérivé halogéné utilisé est de préférence le bromure.

Généralement $R^1$ et $R^2$ représentent le même radical alkyle et il n'est pas nécessaire de séparer les produits de la réaction.

Les dithiols de départ sont des produits du commerce ou peuvent être préparés à partir de produits du commerce par des procédés classiques.

Selon l'invention le thio-éther utilisé pour la séparation du palladium peut se trouver soit dans une phase

liquide immiscible à la solution aqueuse, soit fixé sur une phase solide.

Aussi, selon un premier mode de réalisation du procédé de l'invention, le thio-éther est dissous ou dilué dans un solvant organique, et l'on met en contact la solution aqueuse contenant le palladium avec le solvant organique contenant le thio-éther, puis on sépare la solution aqueuse appauvrie en palladium du solvant organique contenant le complexe de palladium.

Le solvant ou diluant organique utilisé peut être choisi, par exemple, parmi les solvants chlorés tels que $CHCl_3$, $CH_2Cl_2$, $CCl_3CH_3$, $CHCl_2CHCl_2$, $ClCH_2CH_2Cl$ et le dichlorobenzène, l'éther, les hydrocarbures tels que l'heptane, le dodécane, le benzène et les alkylbenzénes, et le benzonitrile.

La concentration en thio-éther du solvant organique peut varier dans une large gamme et dépend en particulier du solvant ou diluant organique utilisé.

En effet, cette concentration doit être telle que l'on obtienne une solution organique homogène sans cristallisation du thio-éther ou des complexes thio-éther-Pd. Généralement, on utilise une concentration en thioéther allant de 0,5 à 25% (P/V).

Lorsque le ligand est liquide et suffisamment lipophile pour être insoluble dans la phase aqueuse, celui-ci peut être utilisé sans solvant.

Dans ce premier mode de réalisation du procédé de l'invention, la mise en contact des deux phases liquides et leur séparation peuvent être effectuées dans des appareillages classiques, par exemple dans des colonnes d'échange à co-courant ou à contre-courant telles que les colonnes pulsées ou dans des mélangeurs-décanteurs. On opère généralement à la pression et à la température ambiantes.

Généralement, la solution aqueuse de départ a une concentration en acide nitrique de 0,1 à 5mol/l.

On peut ensuite réextraire le palladium dans une solution aqueuse, par exemple dans de l'eau.

Selon un second mode de réalisation du procédé de l'invention, le ligand thio-éther est supporté par une phase solide ; dans ce cas, on met en contact la solution aqueuse contenant le palladium avec la phase solide supportant le thio-éther et on sépare la solution aqueuse appauvrie en palladium de la phase solide qui comprend le complexe de palladium.

Les phases solides utilisables sont par exemple des phases organiques ou minérales greffées avec le motif

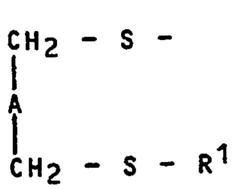

ou

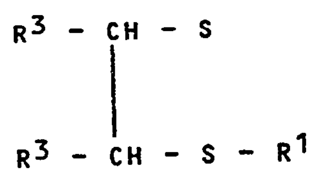

A titre d'exemple, les phases organiques utilisables peuvent être en particulier des polymères de dérivés du styrène tels que le polyméthylstyrène comportant sur ses groupes méthyle le radical de formule Ia ou IIa. Cette phase solide peut être préparée en faisant réagir le polychlorométhylstyrène avec un thiol de formule :

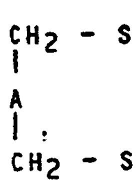

ou

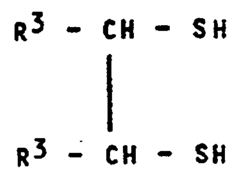

dans un solvant organique, en présence d'un iodure d'alkyle ; ceci correspond au schéma réactionnel suivant :

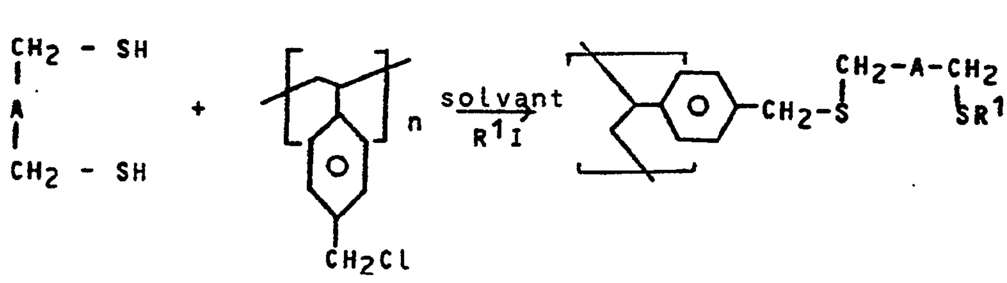


Comme on l'a vu précédemment, le procédé de l'invention s'applique en particulier à la récupération du palladium présent dans les solutions aqueuses nitriques provenant du retraitement des combustibles nucléaires irradiés, par exemple aux solutions aqueuses de dissolution des éléments combustibles nucléaires irradiés.

D'autres caractéristiques et avantages de l'invention apparaîtront mieux à la lecture des exemples suivants donnés bien entendu à titre illustratif et non limitatif, en référence au dessin annexé sur lequel

- la figure 1 représente les spectres UV/visible de solutions aqueuses nitriques après extraction du palladium par le procédé de l'invention,
- la figure 2 représente les spectres d'absorption UV/visible de solutions de $Pd^{2+}$, et
- la figure 3 est un diagramme représentant un mode de mise en oeuvre du procédé de l'invention appliqué au traitement d'une solution de dissolution de combustibles irradiés.

**Exemples 1** : Préparation du trithia-6,9,12 heptadécane. (Composé n° 1) de formule :

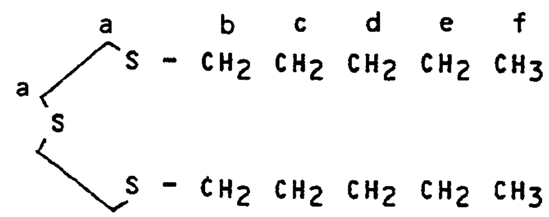

Dans un tricol de 500ml, sous argon, muni d'un thermomètre, d'un réfrigérant et d'une ampoule à brome, on introduit 150ml d'éthanol absolu. On y dissout 4,6g de sodium (0,20mol) et on chauffe à 45°C. On ajoute ensuite lentement une solution éthanolique de 15,43g de thia-3 pentanedithiol1,5 (0,10mol). Après avoir agité pendant 30min, on additionne lentement une solution éthanolique de 25,58g de chloro-1 pentane (0,24mol) et on porte à reflux pendant 4h. On ramène le mélange réactionnel à la température ambiante et on filtre. On obtient ainsi 28,8g de produit brut que l'on solubilise dans 200ml de dichlorométhane et on lave le tout avec 2x100ml d'eau distillée. On sèche sur $MgSO_4$ et on filtre. Après évaporation du solvant, on recristallise deux fois dans l'éthanol et on obtient ainsi 18,21g de trithia-6,9,12 heptadécane, ce qui correspond à un rendement de 62%. Les caractéristiques de ce thio-éther sont les suivantes :

| | |
|---|---|
| - point de fusion : | < 50°C |
| - $^1H$ RMN 200 MHz ($CDCl_3$) : | δ2,75 (m, 8Ha) |
| | 2,55 (t, 4Hb) |
| | 1,60 (q, 4Hc) |
| | 1,35 (m,4Hd et 4He) |
| | 0,90 (t, 6Hf) |

**Exemple 2** : Préparation du trithia-11,14,17 heptacosane (composé n° 2) de formule :

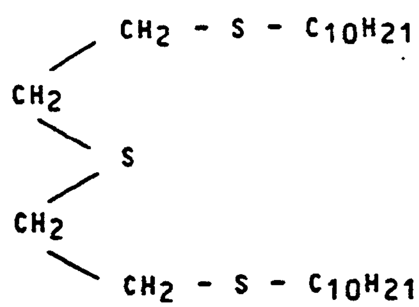

Composé n° 2

On suit le même mode opératoire que dans l'exemple 1 pour préparer le composé n° 2 sauf que l'on utilise 42,42g de chloro-1 décane (0,24mol) au lieu des 25,58g de chloro-1 pentane.

On obtient le composé n° 2 avec un rendement de 96%.

<u>**Exemple 3**</u> : <u>Préparation du dithia-6,10 pentadécane</u> (composé n° 3) de formule :

$$CH_2 - S - C_5H_{11}$$
$$|$$
$$CH_2$$
$$|$$
$$CH_2 - S - C_5H_{11}$$

On suit le même mode opératoire que dans l'exemple 1 pour préparer ce composé n° 3 sauf que l'on utilise 10,8g de propanedithiol-1,3 (0,10mol)au lieu des 15,43g de thia-3 pentanedithio-1,5.

On obtient le composé n° 3 avec un rendement de 73%.

<u>**Exemple 4.**</u>

Dans cet exemple on utilise le composé n° 1 de l'exemple 1, soit le trithia-6,9,12 heptadécane dans une solution chloroformique comprenant 1% (en P/V) du composé n° 1 pour extraire le palladium à partir d'une solution nitrique.

On met en contact 1ml d'une solution de chlorure de palladium $PdCl_2$ à 0,4g/l ($2{,}25.10^{-3}$mol/l) dans de l'acide nitrique 1N, avec 1ml de la solution chloroformique contenant 1% (en P/V) du composé n° 1. Après 5min d'agitation, on laisse décanter les deux phases et on remarque que les deux phases sont colorées alors qu'au départ seule la solution aqueuse qui contenait le sel de palladium était colorée.

On constate ainsi que la solution chloroformique du composé n°1 permet d'extraire le palladium présent dans la solution aqueuse.

<u>**Exemple 5.**</u>

Dans cet exemple on utilise le composé n° 2 et on suit le même mode opératoire que dans l'exemple 4 pour extraire le palladium à partir de la même solution aqueuse nitrique au moyen d'une solution chloroformique à 1% (P/V) du composé n°2 de l'exemple 2.

On constate également qu'après décantation, la coloration est répartie dans les deux phases, ce qui montre que le palladium a été extrait dans la phase organique.

<u>**Exemple 6.**</u>

Dans cet exemple on utilise le composé n° 3 de l'exemple 3, et on suit le même mode opératoire que dans l'exemple 4 pour extraire le palladium à partir de la même solution aqueuse. Après décantation des deux phases, on constate que celles-ci sont toutes deux colorées, ce qui montre que le palladium a été extrait dans la phase organique.

<u>**Exemple comparatif 1.**</u>

Dans cet exemple on utilise le dithia-6,9-tétradécane, soit le composé de formule :

$$CH_2 - S - C_5H_{11}$$
$$|$$
$$CH_2 - S - C_5H_{11}$$

pour extraire le palladium à partir de la même solution aqueuse nitrique que celle des exemples 4 à 6. On suit le même mode opératoire mais dans ce cas après décantation des deux phases, on constate que seule la phase aqueuse est colorée. Ainsi le palladium n'est pas extrait par ce thio-éther car ce dernier ne possède pas une forte lipophilicité comme ceux utilisés dans les exemples 4, 5 et 6.

<u>**Exemples 7 à 12.**</u>

Dans ces exemples on évalue la sélectivité pour l'extraction du palladium, du thioéther (composé n° 1) utilisé dans l'exemple 4, en suivant le même mode opératoire que dans l'exemple 4 avec d'autres solutions nitriques colorées de sels métalliques.

On évalue visuellement le degré d'extraction comme dans l'exemple 4, après 5min d'agitation et après 48h.

La composition des solution aqueuses de départ et les résultats obtenus sont donnés dans le tableau 1.

Tableau 1

| Ex. | Solution aqueuse de départ | Extraction | |
|---|---|---|---|
| | | après 5min | après 48h |
| 4 | $PdCl_2$ 0,4g $l^{-1}$, $HNO_3$ 1N | oui | oui |
| 7 | $UO_2(NO_3)_2$ 250g $l^{-1}$, $HNO_3$ 1 N | non | non |
| 8 | $HAuCl_4$, $HNO_3$ 1N | non | non |
| 9 | $FeCl_3$, $HNO_3$ 1N | non | non |
| 10 | $CuCl_2$, $HNO_3$ 1N | non | non |
| 11 | $NiCl_2$, $HNO_3$ 1N | non | non |
| 12 | $RuCl_3$, $HNO_3$ 1N | non | non |

Au vu de ces résultats on constate que tous les autres métaux, notamment l'uranium, ne sont pas extraits par ce thio-éther. Ainsi ce ligand permet l'extraction du palladium mais n'a pas d'affinité pour les sels de fer, de cuivre, de nickel, de ruthénium et surtout d'uranium.

**Exemples 13 à 18.**

Dans ces exemples on étudie l'influence de la concentration en acide nitrique de la solution aqueuse de départ sur l'extraction du palladium.

Dans tous les cas, on met en contact 5ml d'une solution aqueuse nitrique contenant 0,4g/l, (soit 2,25mol/l de $PdCl_2$) avec 5ml d'une solution du ligand de l'exemple 1 à une concentration de 10g/l dans le chloroforme. On mélange les deux solutions pendant 1min, puis on laisse décanter pendant une demi-heure et on détermine ensuite la densité optique des phases organique et aqueuse par spectrométrie UV/visible.

Les spectres UV/visible des solutions aqueuses avant extraction présentent un maximum à 420nm alors que ces solutions présentent, après extraction, un maximum à 370nm. Le maximum à 370nm peut être attribué à la présence de palladium uniquement complexé sous la forme ($Pd^{2+}$ (composé n° 1) $(NO_3)_2$) car on obtient en effet le même type de spectre en saturant une solution de $Pd^{2+}$ à 0,04g/l dans de l'acide nitrique 0,1N avec des cristaux du composé n° 1 utilisé dans cet exemple.

Dans le tableau 2, sont indiquées les longueurs d'onde λmax correspondant au maximum d'absorption des solutions aqueuses, avant et après extraction, ainsi que les densités optiques DO correspondant à la longueur d'onde maximale λmax, avant et après extraction.

Tableau 2

| Exemple | 13 | 14 | 15 | 16 | 17 |
|---|---|---|---|---|---|
| $(HNO_3)$ en mol.$l^{-1}$ | 0,1 | 0,2 | 0,5 | 1,0 | 2,0 |
| λmax (nm) avant extraction | 420 | 420 | 420 | 420 | 420 |
| DO λmax avant extraction | 0,482 | 0,433 | 0,450 | 0,442 | 0,471 |
| λmax (nm) après extraction | 370 | 370 | 370 | 360 | 350 |
| DO λmax après extraction | 0,446 | 0,386 | 0,285 | 0,388 | 0,652 |

La figure 1 représente les spectres UV visibles des solutions aqueuses après extraction ; la courbe en pointillé se rapporte à la solution aqueuse 1N avant extraction.

Le déplacement du maximum d'absorption et l'augmentation de celle-ci pour les concentrations en acide nitrique supérieure à 0,5N peut s'expliquer par le fait que la solubilité dans la solution aqueuse du ligand thioéther et/ou du complexe correspondant augmente avec l'acidité de cette solution.

A partir des résultats obtenus dans les exemples 13 à 17, on peut calculer les coefficients de partage du palladium pour des concentrations en $HNO_3$ peu élevées et calculer ensuite la constante d'extraction. Les coefficients de partage du palladium Dpd qui correspondent au rapport de la concentration en Pd de la phase organique $(Pd)_0$ sur la concentration en palladium $(Pd)_a$ de la phase aqueuse sont donnés dans le tableau 3 qui suit.

Tableau 3

| Exemple | 13 | 14 | 15 |
|---|---|---|---|
| $(HNO_3)$ (mol/l) | 0,1 | 0,2 | 0,5 |
| DO observée en phase aqueuse | 0,446 | 0,386 | 0,285 |
| $D_{Pd}$ | 20,83 | 24,4 | 32,25 |

Au vu de ces résultats, on remarque que le coefficient de partage est très important.

On peut effectuer le calcul des constantes d'extraction en partant des densités optiques observées dans la phase organique, avant et après extraction, en comparant les densités optiques obtenues pour des concentrations initiales en acide nitrique de 0,1N et > 0,5N. Pour cette dernière, on considère que le palladium est totalement extrait, ce qui permet de déterminer le coefficient d'extinction moléculaire du complexe et par conséquent le coefficient de partage et la constante d'extraction du palladium, selon la formule :

$$K_{ex} = \frac{(PdL(NO_3)_2)_0}{(Pd)\ (NO_3)^2\ (L)_0}$$

où L représente le ligand thio-éther, en estimant que la concentration en ligand à l'équilibre est proche de la concentration initiale en ligand.

Les résultats obtenus dans le cas de l'exemple 13 sont donnés dans le tableau 4 qui suit.

**Tableau 4**

| | $\lambda$ = 440 nm | $\lambda$ = 450 nm |
|---|---|---|
| $D_{Pd}$ | 1,06 | 1,004 |
| $K_{ex}(mol^{-3}\ l^3)$ | $3{,}4\ 10^5$ | $3{,}24\ 10^5$ |

Les constantes d'extraction obtenues montrent que ce thio-éther est un extractant particulièrement efficace du palladium.

**Exemple 18** : Réextraction du palladium.

Dans cet exemple on extrait tout d'abord le palladium dans une solution chloroformique contenant 1% (en P/V) du thio-éther (composé n° 1) de l'exemple 1 à partir d'une solution nitrique 0,1N contenant $2{,}25.10^{-3}$mol/l de $PdCl_2$.

On réalise l'extraction en mettant en contact 5ml de la solution aqueuse avec 5ml de la solution organique, sous agitation pendant 1min, et en laissant ensuite décanter pendant une demi-heure afin de récupérer une phase aqueuse appauvrie en palladium et une phase organique contenant le complexe de palladium.

On met ensuite en contact, sous agitation, pendant 5min, 2ml de la phase organique séparée avec 18ml de $H_2O$, et on laisse ensuite décanter pendant 2h. On détermine alors la quantité de palladium réextraite dans l'eau par mesure de la densité optique en UV/visible. Le spectre UV visible obtenu est représenté sur la figure 2.

Sur cette figure, on a représenté également en pointillé le spectre UV/visible d'une solution à $0{,}225.10^{-3}$mol/l de $PdCl_2$ dans $HNO_3$ 0,01N, saturée du composé n° 1. La présence d'un maximum à 370nm indique que c'est le complexe $PdL(NO_3)_2$ qui est réextrait. La densité optique mesurée à cette longueur d'onde permet de déterminer le rendement de l'opération d'extraction-réextraction.

$$Y\ \% = \frac{DO^{370}_{\text{réextrait}} \times 9}{DO^{370}_{0{,}225.10^{-3}mol.l^{-1}} \times 10} \simeq 100\%$$

L'extraction et la réextraction par l'eau du palladium peuvent donc être effectuées quantitativement en utilisant le composé n° 1.

**Exemple 19.**

Dans cet exemple, on utilise le ligand thio-éther (composé n° 1) de l'exemple 1 pour extraire le palladium

à partir d'une solution aqueuse contenant du palladium et de l'uranium, ayant la composition suivante :
$HNO_3$ : 1N
$PdCl_2$ : 0,4g/l (2,25.10$^{-3}$mol/l)
$UO_2^{2+}$ : 250g/l.

Dans cet exemple on met en contact 5ml de la solution aqueuse avec 5ml d'une solution chloroformique contenant 1% (P/V) du composé n° 1 de l'exemple 1. On agite pendant 1min, puis on laisse décanter pendant une demi-heure. On sépare alors la phase aqueuse de la phase organique et on met ensuite 2ml de la phase organique en contact avec 18ml d'eau pendant 5min et on laisse décanter pendant 2h. On sépare ensuite la phase aqueuse et l'on mesure sa densité optique par spectrométrie UV/visible. Le spectre UV/visible de la solution aqueuse de réextraction présente les mêmes caractéristiques que celui obtenu dans l'exemple précédent. Ainsi il présente un maximum d'absorption à 370nm et peu ou pas d'absorption due au nitrate d'uranyle. A partir de la densité optique observée à 370nm, on peut déterminer le rendement de l'opération d'extraction et de réextraction (Y). Celui-ci est égal à 99,5%.

Ainsi, la présence d'une grande quantité de nitrate d'uranyle n'affecte que très peu l'efficacité de l'extraction par le composé n° 1 et peu ou pas la sélectivité de celle-ci.

**Exemple 21.**

Dans cet exemple on utilise le thio-éther (composé n° 1) de l'exemple 1 pour extraire le palladium présent dans une solution active contenant de l'uranium, du plutonium et des produits de fission, et ayant la composition suivante :
$UO_2^{2+}$ : 250g/l (en uranium)
$Pu^{4+}$ : 550mg/l
$Pd^{2+}$ : 15 à 20mg/l
acidité nitrique : 0,9N
activité : 22Ci/l.

On réalise les opérations suivantes d'extraction, de lavage et de réextraction conformément au schéma représenté sur la figure 3.

Ainsi, on met en contact un volume de la solution aqueuse de départ $A_0$ avec un volume d'une solution chloroformique $O_0$ contenant 1% (P/V) du thio-éther (composé n°1) pendant 5min, puis on laisse décanter la phase organique $O_1$ et on la sépare de la phase aqueuse $A_1$. On lave la phase organique $O_1$ par de l'acide nitrique 3N et l'on obtient une phase aqueuse $A_2$ et une phase organique $O_2$. On réextrait le palladium présent dans la phase organique $O_2$ par de l'eau, ce qui permet d'obtenir une phase aqueuse de réextraction $A_3$ et une phase organique $O_3$ qui peut être recyclée en $O_0$.

On détermine les teneurs en uranium, plutonium, palladium et l'activité gamma des phases aqueuses obtenues après extraction, lavage et réextraction. Les résultats obtenus sont donnés dans le tableau 5.

**Tableau 5**

| phase aqueuse | U | Pu | Pd | Activité |
|---|---|---|---|---|
| A1 | 214g | 550mg/l | 0 | 22,3 Ci/l |
| A2 | 25mg/l | 0,7mg/l | 2,1mg/l | 2,5mCi/l |
| A3 | 5mg/l | <0,5mg/l | 13,3mg/l | 0,74mCi/l |

Au vu de ce tableau on constate que l'on extrait le palladium avec un excellent rendement, la seule perte de palladium se trouvant dans la solution aqueuse de lavage. Toutefois, on peut éviter cette perte en utilisant de l'acide nitrique plus dilué dans lequel le complexe thio-éther-$Pd^{2+}$ est moins soluble.

Ainsi, on peut récupérer 70 à 100% du palladium contenu dans une solution de dissolution d'éléments

combustibles irradiés. Les extractants de l'invention permettent donc de réaliser de façon quantitative la récupération du palladium à partir de la solution de dissolution de premier cycle avec un facteur de décontamination de $3.10^4$ après une extraction, un lavage et une réextraction.

**<u>Exemple 22.</u>**

Dans cet exemple, on réalise l'extraction du plutonium en utilisant une phase solide constituée par une résine sur laquelle est fixée un radical de formule Ia dans laquelle $R^1$ représente $CH_3$ et A représente $CH_2$.

a) <u>Préparation de la résine comprenant le thioéther.</u>

Dans un tricol de 50ml muni d'un réfrigérant, d'un thermomètre et d'une ampoule de coulée, on fait réagir 2,16g (20mmol) de $HS-(CH_2)_3-SH$ dans 14ml de diméthylformamide (DMF) avec 4,94g (44mmol) de butylate de potassium et 0,2g ($9,52.10^{-1}$mmol) de bromure de tétraéthylammonium. On laisse sous agitation magnétique pendant 10min, puis on introduit à l'aide de l'ampoule de coulée, 1,2ml (20mmol) d'iodure de méthyle solubilisé dans 1,5ml de diméthylformamide sur une durée d'une heure. On ajoute ensuite 2,86g (2méq.) de polychlorométhylstyrène et on porte le mélange à 100°C pendant 3 jours.

On laisse refroidir le mélange à la température ambiante et on lave deux fois la résine avec 10ml de méthanol et deux fois avec 10ml d'eau permutée. On place alors la résine dans 20ml de diméthylformamide au reflux à 100°C pendant 3 jours.

On laisse refroidir le mélange à la température ambiante et on lave la résine 5 fois avec 10ml de chloroforme, puis on place la résine pendant 24h dans un dessicateur contenant du $P_2O_5$ sous vide.

b) <u>Extraction du palladium.</u>

On met en suspension 1g de la résine précédemment synthétisée qui comprend le thioéther, dans 40ml d'une solution aqueuse nitrique contenant 1mmol/l de palladium et 1,38mol/l d'acide nitrique. On laisse sous agitation magnétique pendant 1h. Au contact de la résine, la solution, qui était jaune au départ, se décolore tandis que la résine devient marron terreux.

On sépare alors la résine de la solution par filtration sur du papier filtre et on détermine la teneur en palladium de la solution nitrique par absorption atomique.

On trouve ainsi que la totalité du palladium a été extraite par la résine, celle-ci contient donc 4,256mg de palladium par gramme de résine.

Ainsi, l'extraction est totale mais la masse de palladium extraite par gramme de résine est faible.

Pour évaluer la sélectivité de la résine, on réalise le même essai d'extraction mais en utilisant des solutions aqueuses nitriques contenant 1mmol/l de rhodium, de nickel, de cuivre ou d'uranium.

En fin d'opération, on constate que la résine ne contient aucun des métaux présents dans les solutions aqueuses. En effet, le pourcentage d'extraction du rhodium, du nickel, du cuivre et de l'uranium par cette résine est de 0.

Ainsi, la résine présente une très bonne sélectivité pour le palladium.

## Revendications

1. Procédé pour séparer le palladium présent dans une solution aqueuse, caractérisé en ce qu'il comprend les étapes suivantes :

   a) mettre en contact la solution aqueuse contenant le palladium avec un extractant organique constitué par un thioéther répondant à la formule :

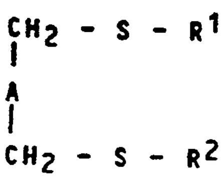 (I) ou 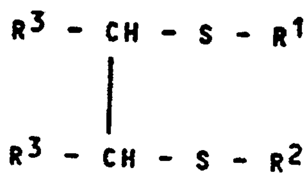 (II)

   ou comprenant un radical de formule :

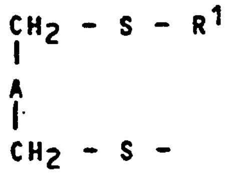 (I) ou 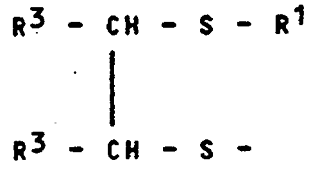 (II a)

dans lesquelles $R^1$ et $R^2$ qui peuvent être identiques ou différents, représentent des radicaux alkyle, $R^3$ représente un radical alkyle et A représente un radical bivalent choisi parmi les radicaux de formule :

$-(CH_2)_m-$

$-(CH_2)_n-X-(CH_2)_p,$

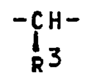

dans lesquelles m est égal à 0 ou est un nombre enrier allant de 1 à 6, n et p sont des nombres entiers allant de 1 à 6 et X représente 0 ou S, pour complexer le palladium avec cet extractant, et
b) séparer la solution aqueuse appauvrie en palladium du complexe de palladium formé dans l'étape a).

2. Procédé selon la revendication 1, caractérisé en ce que le thioéther répond à la formule (I) dans laquelle A représente $-CH_2-$, et $R^1$ et $R^2$ représentent le radical pentyle.

3. Procédé selon la revendication 1, caractérisé en ce que le thioéther répond à la formule (I) dans laquelle A représente $-CH_2-S-CH_2-$, et $R^1$ et $R^2$ représentent le radical pentyle.

4. Procédé selon la revendication 1, caractérisé en ce que le thioéther répond à la formule (I) dans laquelle A représente $-CH_2-S-CH_2-$, et $R^1$ et $R^2$ représentent le radical décyle.

5. Procédé selon l'une quelconque des revendication 1 à 4, caractérisé en ce que le thio-éther est dissous ou dilué dans un solvant organique, et en ce que l'on met en contact la solution aqueuse contenant le palladium avec le solvant organique contenant le thio-éther, puis on sépare la solution aqueuse appauvrie en palladium du solvant organique contenant le complexe de palladium.

6. Procédé selon l'une quelconque des revendications 1 à 4, caractérisé en ce que le thio-éther est supporté par une phase solide, et en ce que l'on met en contact la solution aqueuse contenant le palladium avec la phase solide supportant le thio-éther.

7. Procédé selon la revendication 6, caractérisé en ce que la phase solide est du polym"thylstyrène comportant sur ses groupements méthyle un radical de formule (Ia) ou (IIa).

8. Procédé selon l'une quelconque des revendications 1 à 7, caractérisé en ce que la solution aqueuse est une solution nitrique provenant du retraitement de combustibles nucléaires irradiés.

9. Procédé selon la revendication 8, caractérisé en ce que la solution nitrique est une solution de dissolution d'éléments combustibles nucléaires irradiés.

10. Procédé selon l'une quelconque des revendications 1 à 9, caractérisé en ce que la concentration en acide nitrique de la solution aqueuse est de 0,1 à 5mol/l.

11. Procédé selon la revendication 5, caractérisé en ce que l'on récupère ensuite le palladium présent dans le solvant organique par réextraction par une solution aqueuse.

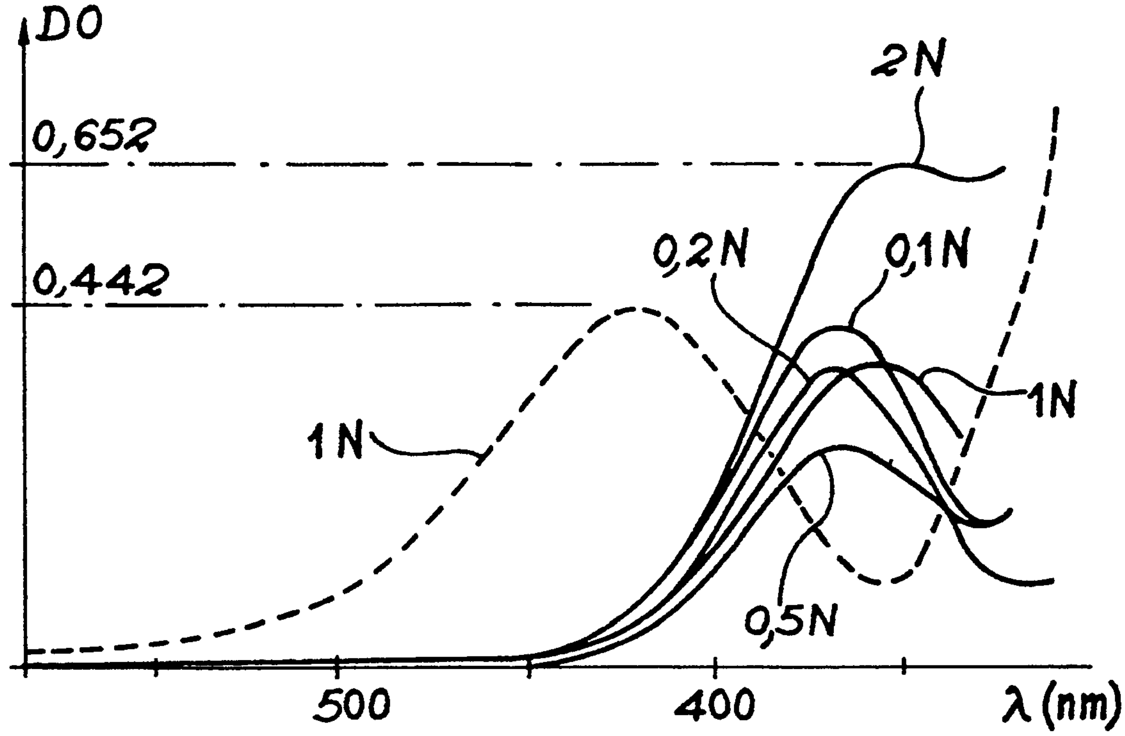


FIG. 1

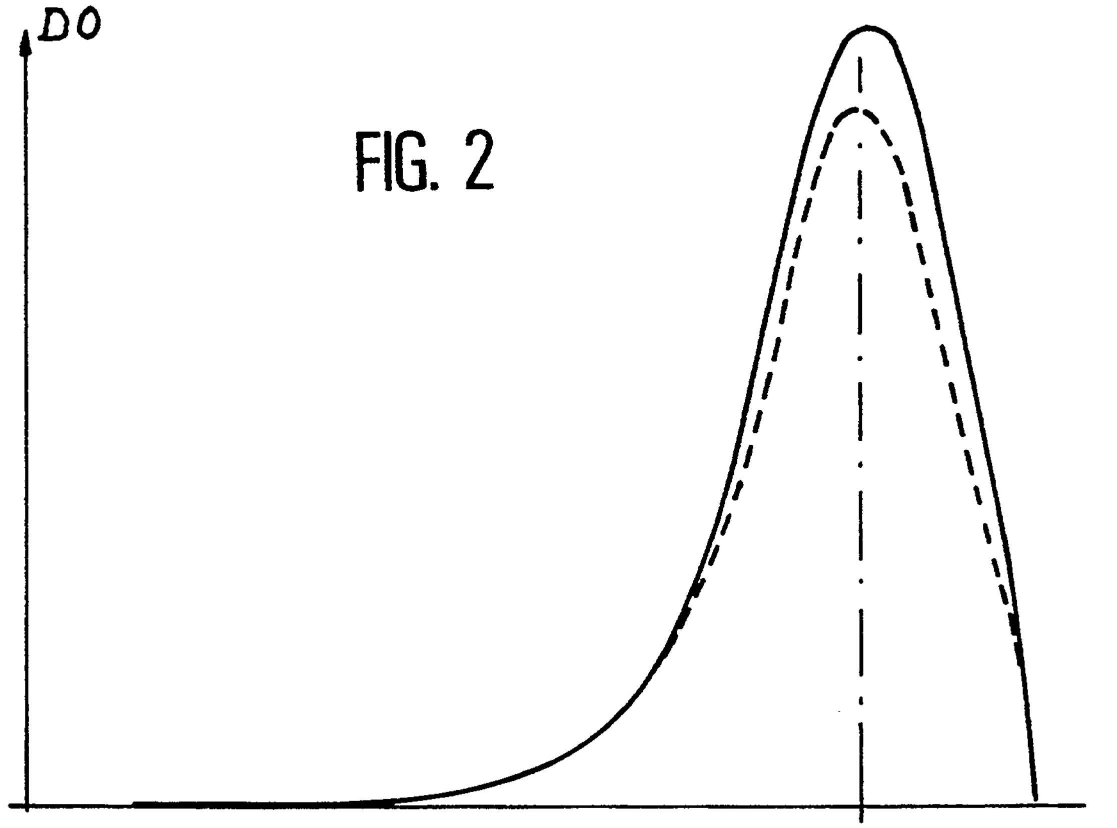


FIG. 2

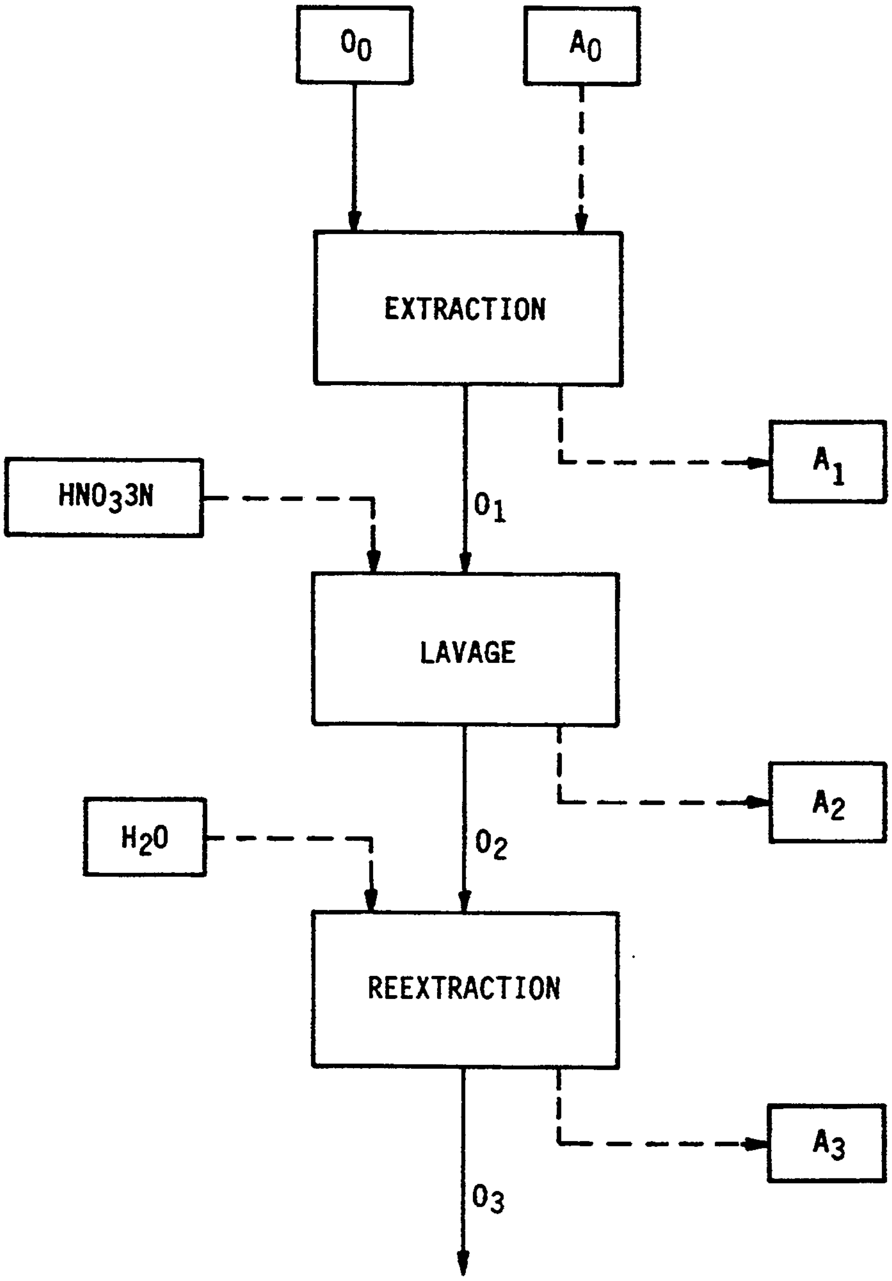
$O_0$
$A_0$
EXTRACTION
$A_1$
$HNO_3$3N
$O_1$
LAVAGE
$A_2$
$H_2O$
$O_2$
REEXTRACTION
$A_3$
$O_3$

FIG. 3

Office européen des brevets

# RAPPORT DE RECHERCHE EUROPEENNE

Numero de la demande

EP 91 40 1242

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | SOVIET ATOMIC ENERGY, vol. 64, no. 2, février 1988, pages 119-126, New York, US; V.S. SHMIDT et al.: "Palladium in spent power station fuel solvent-extraction reprocessing" * Pages 123,124 * | 1-11 | C 22 B 3/00<br>G 21 C 19/46<br>G 21 C 19/50<br>C 07 C 321/14 |
| A | JOURNAL OF INORGANIC & NUCLEAR CHEM., vol. 42, no. 6, 1980, pages 885-889, Pergamon Press, Ltd, Oxford, GB; D. SEVDIC et al.: "Macrocyclic polythiaethers as solvent extraction reagents - III" | 1-11 | |
| A | CHEMICAL ABSTRACTS, vol. 72, no. 16, 1970, page 32, abrégé no. 79858d, Columbus, Ohio, US; & JP-A-69 26 196 (MITSUBISHI RAYON CO., LTD) 04-11-1969 * Ligne 12 * | 1 | |
| A | US-A-3 315 000 (D.L. RANSLEY) * Tableau II, lignes 3-6; tableau III, lignes 4-7 * | 1 | DOMAINES TECHNIQUES RECHERCHES (Int. Cl.5) |
| A | CHEMICAL ABSTRACTS, vol. 85, no. 19, 1976, page 481, abrégé no. 142536h, Columbus, Ohio, US; R.V. ZAINULLINA et al.: "Oxidation of bis(sulfides) from mercaptans of different structure", & NEFTEKHIMIYA 1976, 16(2), 293-8 * Abrégé * | 1 | C 22 B 3/00<br>G 21 C 19/00<br>C 07 C 321/00 |

-/-

Le présent rapport a été établi pour toutes les revendications

| Lieu de la recherche | Date d'achèvement de la recherche | Examinateur |
|---|---|---|
| LA HAYE | 23-08-1991 | SANCHEZ Y GARCIA J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 2

Office européen des brevets

# RAPPORT DE RECHERCHE EUROPEENNE

Numero de la demande

EP 91 40 1242

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 58, no. 7, 1963, abrégé no. 6684d, Columbus, Ohio, US; R. SASIN et al.: "Addition of alkanedithiols to olefins", & J. CHEM. ENG. DATA 8, 102-3(1963) * Abrégé * --- | 1 | |
| A | DE-A-2 154 318 (HENKEL) * Page 20, tableau, exemples 51-53; revendication 1 * --- | 1 | |
| A | EP-A-0 177 784 (PENNWALT) * Exemples 3,6 * --- | 1 | |
| A | GB-A- 966 929 (ESSO) * Exemples 7,8,9 * --- | 1 | |
| A | CHEMICAL ABSTRACTS, vol. 85, no. 19, 1976, page 481, abrégé no. 142531c, Columbus, Ohio, US; G.M. PROKHOROV et al.: "Bis(sulfides) from mercaptans of Ishimbai petroleums", & NEFTEKHIMIYA 1976, 16(2), 285-92 * Abrégé * --- | 1 | DOMAINES TECHNIQUES RECHERCHES (Int. Cl.5) |
| A | SYNTHESIS, no. 9, septembre 1987, pages 841-843, Stuttgart, DE; E. ANKLAM: "Synthese von alpha-Halogen-omega-alkylthio-alkanen und alpha-omega-Bisalkylthio-alkanen" * Page 843, tableau 2 * --- | 1 | |
| A | US-A-2 519 586 (D.A. McCAULAY) * Colonne 8, exemple 1 * --- | 1 | |
| A | US-A-3 293 209 (F.P. BALDWIN et al.) * Exemples 5,7,8,9 * --- -/- | 1 | |

Le présent rapport a été établi pour toutes les revendications

| Lieu de la recherche | Date d'achèvement de la recherche | Examinateur |
|---|---|---|
| LA HAYE | 23-08-1991 | SANCHEZ Y GARCIA J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 3

Office européen des brevets

# RAPPORT DE RECHERCHE EUROPEENNE

Numero de la demande

EP 91 40 1242

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 021 246 (P.L. BACHMAN et al.)<br>* Revendication 1 *<br>--- | 1 | |
| A | US-A-3 242 135 (D.E. BOWN et al.)<br>* Colonne 9, lignes 9,10 *<br>----- | 1 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 23-08-1991 | SANCHEZ Y GARCIA J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)